# EUROPEAN PATENT APPLICATION

(11) **EP 4 685 229 A1**
(43) Date of publication of application: **28.01.2026**
(21) Application number: 24775025.0
(22) Date of filing: 22.03.2024
(51) Int. Cl.: C12N 5/0775, A61L 27/24, A61L 27/38, C12N 5/02

(54) **PERIODONTAL TISSUE REGENERATION MATERIAL**

(30) Priority: 22.03.2023 JP 2023045232
(71) Applicant: Cyfuse Biomedical K. K., Tokyo 108-6301 (JP); Hiroshima University, Higashihiroshima-shi Hiroshima 739-8511 (JP)
(72) Inventor: KAJIYA Mikihito, Higashihiroshima-shi, Hiroshima 739-8511 (JP); YOSHII Hiroki, Higashihiroshima-shi, Hiroshima 739-8511 (JP); YOSHINO Mai, Higashihiroshima-shi, Hiroshima 739-8511 (JP); MAEKAWA Toshihiko, Tokyo 108-6301 (JP); TORII Yoko, Tokyo 108-6301 (JP); TAKAI Harumi, Tokyo 108-6301 (JP)
(74) Representative: Mewburn Ellis LLP
(86) International application number: PCT/JP2024/080036
(87) International publication number: WO 2024/195894

(57) **Abstract**

There is a demand for establishing a technique for realizing tissue regeneration even in cases of severe periodontal tissue loss. The present invention provides a periodontal tissue regeneration material for grafting, which contains graft tissue containing mesenchymal stem cells and collagen secreted from the mesenchymal stem cells. The graft tissue each include 2 × 10⁵ or more cells, and have a projected area equivalent circle diameter or a thickness of 1 mm or more.

## Description

### TECHNICAL FIELD

The present invention relates to a periodontal tissue regeneration material for grafting, which contains mesenchymal stem cells (mesenchymal stromal cells) and collagen secreted by the mesenchymal stem cells. The present invention also relates to a method for producing a cellular construct for grafting, the method comprising fabricating cell clumps (C-MSCs) using mesenchymal stem cells and extracellular matrix secreted by the mesenchymal stem cells, and using these C-MSCs to fabricate the cellular construct for grafting.

### BACKGROUND ART

Periodontal disease is the collective term for gingivitis and periodontitis. Gingivitis is the early stage of periodontal disease, and it becomes periodontitis as the condition progresses. Periodontitis is an inflammatory disease that causes destruction of periodontal tissue, which consists of the alveolar bone, periodontal ligament, and cementum supporting the teeth, due to the host's immune response to bacterial infection. As periodontitis progresses, vertical bone defect may occur, in which the alveolar bone is lost vertically along the root of the tooth. Vertical bone defect is classified as one-walled, two-walled, three-walled, or four-walled defect according to the number of remaining walls surrounding the root of the tooth. In addition, horizontal bone defect may occur, which consists of horizontal loss in the height of the alveolar bone.

The basic treatment of periodontitis is to eliminate periodontopathogenic bacteria, suppress inflammation, and stop the progression of periodontal tissue destruction. However, these treatment methods rarely regenerates the lost periodontal tissue. Severe periodontal tissue destruction leaves the tooth unable to support physiological occlusal forces, resulting not only in tooth movement and masticatory disorders, but also in traumatic inflammation, which eventually leads to recurrence of periodontitis and tooth loss. In addition, periodontitis is an exacerbating factor of diabetes and cardiovascular diseases, and tooth loss is a cause of frailty in the elderly. Therefore, there is a need to establish periodontal tissue regeneration therapy for the complete cure of periodontitis.

One conventional periodontal tissue regeneration technique is the cytokine therapy which involves the administration of bFGF. However, since this cytokine therapy activates the self-repair capacity of the remaining cells, mild to moderate tissue destruction, such as class I furcation lesions, class II furcation lesions, and localized vertical bone defect (two- or three-walled bone defects), can be treated, but severe periodontal tissue destruction, such as class III root furcation lesions, extensive bone defect (one-walled bone defects), and horizontal bone defect, is difficult to treat due to the limited number of remaining cells.

In hopes of achieving therapeutic effects even at the stage of severe periodontal tissue destruction, the present inventors have fabricated clumps of mesenchymal stem cells (hereinafter sometimes referred to as "bone marrow-derived C-MSCs") composed of bone marrow-derived mesenchymal stem cells and an extracellular matrix (ECM) protein produced by these cells in Non-patent literature 1, and autologously grafted 48 bone marrow-derived C-MSCs into beagle dogs employed as class III root furcation defect models. As a result, effective periodontal tissue regeneration was induced.

In addition, when the present inventors investigated the mechanism of periodontal tissue curing by C-MSCs grafting in Non-patent literature 2 and 3, they found that the grafted mesenchymal stem cells themselves differentiated appropriately while utilizing type I collagen (COLl) that shapes a cell aggregate as a microenvironment, thereby achieving tissue reconstruction.

### CITATION LIST

[Non-patent literature 1] M. Takewaki, M. Kajiya, K. Takeda, S. Sasaki, S. Motoike, N. Komatsu, S. Matsuda, K. Ouhara, N. Mizuno, T. Fujita, and H. Kurihara, "MSC/ ECM Cellular Complexes Induce Periodontal Tissue Regeneration", Journal of Dental Research, 2017 Aug; 96(9): 984-991.
[Non-patent literature 2] Souta Motoike, Mikihito Kajiya, Nao Komatsu, Susumu Horikoshi, Tomoya Ogawa, Hisakatsu Sone, Shinji Matsuda, Kazuhisa Ouhara, Tomoyuki Iwata, Noriyoshi Mizuno, Tsuyoshi Fujita, Makoto Ikeya, and Hidemi Kurihara, "Clumps of Mesenchymal Stem Cell/Extracellular Matrix Complexes Generated with Xeno-Free Conditions Facilitate Bone Regeneration via Direct and Indirect Osteogenesis", Int. J. Mol. Sci. 2019, 20(16), 3970
[Non-patent literature 3] Hisakatsu Sone, Mikihito Kajiya, Katsuhiro Takeda, Shinya Sasaki, Susumu Horikoshi, Souta Motoike, Shin Morimoto, Hiroki Yoshii, Mai Yoshino, Tomoyuki Iwata, Kazuhisa Ouhara, Shinji Matsuda, and Noriyoshi Mizuno, "Clumps of mesenchymal stem cells/extracellular matrix complexes directly reconstruct the functional periodontal tissue in a rat periodontal defect model", J Tissue Eng Regen Med. 2022 Oct; 16 (10): 945-955

### SUMMARY OF INVENTION

Under these circumstances, there is a need to establish a technique capable of achieving tissue regeneration, even in cases of more severe periodontal tissue defect.

The present invention was made in consideration of the above circumstances, and provides a periodontal tissue regeneration material and the like, as described below.

[1] A periodontal tissue regeneration material for grafting, comprising graft tissue containing mesenchymal stem cells and collagen secreted by the mesenchymal stem cells, wherein
   the graft tissue each has 2 × 10⁵ or more cells and has a projected circular area-equivalent diameter or thickness of 1 mm or more.

As well as the periodontal tissue regeneration material according to [1] above, the present invention also encompasses periodontal tissue regeneration materials according to [1-1]-[1-3] below.
[1-1] A periodontal tissue regeneration material for grafting, comprising graft tissue consisting of mesenchymal stem cells and collagen secreted by the mesenchymal stem cells, wherein
   the graft tissue each has 2 × 10⁵ or more cells and has a projected circular area-equivalent diameter or thickness of 1 mm or more.
[1-2] A periodontal tissue regeneration material for grafting, comprising mesenchymal stem cells and collagen secreted by the mesenchymal stem cells, wherein
   each graft tissue has 2 × 10⁵ or more cells and has a projected circular area-equivalent diameter or thickness of 1 mm or more.
[1-3] A periodontal tissue regeneration material for grafting, consisting of mesenchymal stem cells and collagen secreted by the mesenchymal stem cells, wherein
   each graft tissue has 2 × 10⁵ or more cells and has a projected circular area-equivalent diameter or thickness of 1 mm or more.
[2] The periodontal tissue regeneration material according to [1] above, wherein the graft tissue each contains 2 x 10⁵ ng or more of collagen, and the ratio of the number of cells present in the graft tissue (cells) to the collagen content in the graft tissue (ng) (cells/ng) is 1.0-10.0.
[3] The periodontal tissue regeneration material according to [1] or [2] above, wherein the graft tissue each has 4 × 10⁵ or more cells and has a projected circular area-equivalent diameter or thickness of 1 mm or more.
[4] The periodontal tissue regeneration material according to any one of [1]-[3] above, wherein the graft tissue each contains 2.5 × 10⁵ ng or more of collagen, and the ratio (cells/ng) is 1.6-6.0.
[5] A periodontal tissue regeneration material for grafting, comprising graft tissue containing mesenchymal stem cells and collagen secreted by the mesenchymal stem cells, wherein
   the graft tissue has 50-300 cells per 150 µm × 150 µm of a stained section image and has a projected circular area-equivalent diameter or thickness of 1 mm or more. As well as the periodontal tissue regeneration material according to [5] above, the present invention also encompasses periodontal tissue regeneration materials according to [5-1]-[5-3] below.
[5-1] A periodontal tissue regeneration material for grafting, comprising graft tissue consisting of mesenchymal stem cells and collagen secreted by the mesenchymal stem cells, wherein
   the graft tissue each has 50-300 cells per 150 µm × 150 µm of a stained section image and has a projected circular area-equivalent diameter or thickness of 1 mm or more.
[5-2] A periodontal tissue regeneration material for grafting, comprising mesenchymal stem cells and collagen secreted by the mesenchymal stem cells,
   which has 50-300 cells per 150 µm × 150 µm of a stained section image and has a projected circular area-equivalent diameter or thickness of 1 mm or more.
[5-3] A periodontal tissue regeneration material for grafting, consisting of mesenchymal stem cells and collagen secreted by the mesenchymal stem cells,
   which has 50-300 cells per 150 µm × 150 µm of a stained section image and has a projected circular area-equivalent diameter or thickness of 1 mm or more.
[6] The periodontal tissue regeneration material according to [5] above, wherein the graft tissue has an area fraction occupied by collagen of 40% or more in a COL1-immunohistologically stained section image.
[7] The periodontal tissue regeneration material according to any one of [1]-[6] above, wherein 80% or more of the total cells in the graft tissue are positive for surface antigen markers CD29, CD44, CD73, CD90, CD105 and CD166, and negative for CD34 and CD45.
[8] The periodontal tissue regeneration material according to any one of [1]-[7] above, wherein the periodontal tissue regeneration material has a volume of 1-500 mm³.
[9] The periodontal tissue regeneration material according to any one of [1]-[8] above, wherein the periodontal tissue regeneration material has a roughly cubic shape or a roughly rectangular prism shape.
[10] The periodontal tissue regeneration material according to any one of [1]-[8] above, wherein the periodontal tissue regeneration material is tubular.
[11] The periodontal tissue regeneration material according to any one of [1]-[10] above, wherein the mesenchymal stem cells are at least one selected from the group consisting of bone marrow-derived mesenchymal stem cells, adipose-derived mesenchymal stem cells, placenta-derived mesenchymal stem cells, human iPS cell-derived mesenchymal stem cells, and human ES cell-derived mesenchymal stem cells.
[12] The periodontal tissue regeneration material according to any one of [1]-[11] above, wherein the expression level of at least one YAP/TAZ mechanosignaling-related gene selected from the group consisting of YAP1, TAZ, CTGF, CYR61, RUNX2, BMP2, OPN, and POSTN is twice or more higher than that of the mesenchymal stem cells used as a material.
[13] The periodontal tissue regeneration material according to any one of [1]-[12] above, wherein the collagen is COL1.
[14] A method for producing a cellular construct for grafting, comprising the steps of:
   (a) fabricating cell clumps (C-MSCs) using mesenchymal stem cells and extracellular matrix secreted by the mesenchymal stem cells; and
   (b) arranging or molding the C-MSCs to fabricate the cellular construct for grafting.
[15] The method according to [14] above, wherein, in steps (a) and (b) above, a medium containing ascorbic acid or ascorbic acid phosphate ester is used.
[16] The method according to [14] or [15] above, wherein the C-MSCs have an average particle size of 600-2,000 µm.
[17] The method according to [16] above, wherein the C-MSCs have an average particle size of 800-1,500 µm.
[18] The method according to [17] above, wherein the C-MSCs have an average particle size of 900-1,200 µm.
[19] The method according to any one of [14]-[18] above, wherein in steps (a) and/or (b), shaking culture, agitation culture, or no-shear agitation culture is performed.
[20] A cellular construct for grafting, which is produced by the method according to any one of [14]-[19] above.
[21] The cellular construct for grafting according to [20] above, which is used as a periodontal tissue regeneration material.

Other aspects that can be encompassed by the present invention, for example, include:
a pharmacological composition (pharmaceutical composition) for use in a method for treating periodontitis in a subject, comprising the periodontal tissue regeneration material according to any one of [1]-[13] above;
a pharmacological composition (pharmaceutical composition) for treating periodontitis in a subject, comprising the periodontal tissue regeneration material according to any one of [1]-[13] above;
a method for treating periodontitis, comprising applying the periodontal tissue regeneration material according to any one of [1]-[13] above or the above pharmacological composition (pharmaceutical composition) to a subject;
use of the periodontal tissue regeneration material according to any one of [1]-[13] above for use in the treatment of periodontitis; and
use of the periodontal tissue regeneration material according to any one of [1]-[13] above for the production of an agent (pharmacological material, etc.) for treating periodontitis.

Periodontitis refers to an inflammatory disease that causes destruction of periodontal tissue (alveolar bone, periodontal ligament, and/or cementum) supporting the teeth, due to the host's immune response to bacterial infection, and the above subject includes humans and all other mammals with periodontal tissue.

### EFFECTS OF INVENTION

The periodontal tissue regeneration material of the present invention can be grafted into a site of severe periodontal tissue defect to achieve tissue regeneration at the grafted site.

### BRIEF DESCRIPTION OF DRAWINGS

[Figure 1] A diagram showing an example of a method for producing a periodontal tissue regeneration material of the present invention.
[Figure 2] A particle size distribution diagram of C-MSCs obtained in Example 1.
[Figure 3] An H&E-stained image of a C-MSC and an H&E-stained image of a spheroid composed of mesenchymal stem cells in Example 2.
[Figure 4] DAPI/COL1-immunostained images of a section of C-MSCs and a section of a spheroid composed of mesenchymal stem cells in Example 2.
[Figure 5] Optical microscopic images, H&E-stained images, and DAPI/COL1-immunostained images of a section of the cellular construct of the present invention and a section of C-MSCs, i.e., a comparative subject, in Example 2.
[Figure 6] A diagram showing the expression levels of a YAP/TAZ mechanosignaling-related (bone and periodontal tissue formation-related) gene cluster in the cellular construct of the present invention and C-MSCs, i.e., a comparative subject, in Example 3.
[Figure 7] Images showing the progress in Example 4. (a) A photograph of the cellular construct for grafting being fabricated by a 3D bioprinter, S-PIKE (registered trademark). (b) Photographs of a site of the periodontal tissue defect in a nude rat used in the examination. (c) A photograph of the nude rat immediately after the cellular construct was grafted into the site of the periodontal tissue defect. (d) CT images showing changes over time after grafting.
[Figure 8] H&E-stained images, DAPI-stained (nuclear-stained) images, and human vimentin-stained images of the grafted site 8 weeks after grafting in Example 4.
[Figure 9] Diagrams showing the distribution of the circular-equivalent diameters of C-MSCs of each sample in Example 5.
[Figure 10] Images showing the results of HE staining and DAPI/COL1 staining of a section of each sample in Example 6.
[Figure 11] A diagram showing the expression levels of YAP/TAZ mechanosignaling- and osteogenic differentiation-related genes in Example 7.
[Figure 12] A diagram showing the expression levels of periodontal tissue-related matrix genes in Example 7.
[Figure 13] Images showing the results of periodontal tissue regeneration by grafting three-dimensional cellular constructs (4 weeks after grafting) in Example 8.
[Figure 14] Images showing the results of periodontal tissue regeneration by grafting three-dimensional cellular constructs (8 weeks after grafting) in Example 8.
[Figure 15] An H&E-stained image, a DAPI-stained (nuclear-stained) image, and human vimentin-stained images of the grafted site 8 weeks after grafting in Example 8.

Optical microscopic images, H&E-stained images, and DAPI/COL1-immunostained images of a section of the cellular construct of the present invention and a section of C-MSCs, i.e., a comparative subject, in Example 2.

### DESCRIPTION OF EMBODIMENTS

Hereinafter, the present invention will be described in detail. The scope of the present invention is not limited to the following descriptions, and the present invention may be implemented with modifications other than those illustrated in the following examples without departing from the spirit of the invention. This specification incorporates the entirety of the specification of Japanese Patent Application No. 2023-045232 (filed on March 22, 2023), based on which priority is claimed by this application. In addition, all publications cited herein, including prior art literature, published patent applications, patent publications, and other patent literature, are incorporated herein by reference in their entirety, regardless of their purpose.

### Periodontal tissue regeneration material

One embodiment of the present invention relates to a periodontal tissue regeneration material for grafting, which comprises cell clumps (C-MSCs) containing mesenchymal stem cells and collagen secreted by the mesenchymal stem cells. According to the present invention, the above cell clumps (C-MSCs) are sometimes referred to as graft tissues. The periodontal tissue regeneration material of this embodiment can be produced, for example, by the steps of: (a) fabricating cell clumps (C-MSCs) using mesenchymal stem cells and extracellular matrix, such as collagen, secreted by the mesenchymal stem cells; and (b) arranging or molding the C-MSCs to fabricate a cellular construct.

While any known mesenchymal stem cells can be used, the mesenchymal stem cells are preferably at least one selected from the group consisting of bone marrow-derived mesenchymal stem cells, adipose-derived mesenchymal stem cells, placenta-derived mesenchymal stem cells, human iPS cell-derived mesenchymal stem cells, and human ES cell-derived mesenchymal stem cells, and more preferably at least one selected from the group consisting of bone marrow-derived mesenchymal stem cells and adipose-derived mesenchymal stem cells. Furthermore, the mesenchymal stem cells are preferably human-derived cells, which are either the patient's own cells (autologous) or cells other than the patient's cells (allogeneic). For example, biopsied cells; commercially available frozen cells; cells induced to differentiate from cells derived from a living body, etc. using a reagent, a gene, mRNA, microRNA, etc.; reprogrammed cells; etc., can be used.

Mesenchymal stem cells are cultured or maintained in a medium appropriate for their type and adjusted as needed. Various antibiotics and fetal bovine serum can be added to the medium as necessary, but considering that the medium is intended for grafting into human periodontal tissue, it is preferable to use a xeno-free medium (a medium that does not contain non-human-derived components).

When cell clumps (C-MSCs) are fabricated in step (a), mesenchymal stem cells are allowed to secrete extracellular matrix, such as collagen. For example, mesenchymal stem cells are cultured until they reach confluence, and then continuously cultured to allow the cells to secrete sufficient amount of extracellular matrix, such as collagen. In doing so, the medium preferably contains ascorbic acid or ascorbic acid phosphate ester.

The approximate end of culture can be appropriately determined, for example, by the amount of extracellular matrix, such as collagen, contained in the periodontal tissue regeneration material, i.e., the final product. For example, the amount of C-terminal telopeptide, a product resulting from collagen I synthesis, in the culture supernatant is monitored, and once the level reaches a predetermined value, mesenchymal stem cells and extracellular matrix, such as collagen, are removed from the culture container.

When the removed mesenchymal stem cells and extracellular matrix are placed and cultured in a non-adherent plate in a well-mixed state, spherical cell clumps can be obtained. In doing so, the medium preferably contains ascorbic acid or ascorbic acid phosphate ester.

Herein, spherical cell clumps containing mesenchymal stem cells and extracellular matrix, such as collagen, secreted by the mesenchymal stem cells are sometimes referred to as C-MSCs.

C-MSCs may be of any size, but preferably has an average particle size of 600-2,000 µm, more preferably has an average particle size of 800-1,500 µm, and particularly preferably has an average particle size of 900-1,200 µm. For the average particle size, 50 or more C-MSCs are selected to measure the particle size of the individual C-MSCs and determine their average.

C-MSCs are in a state where cells and collagen are mixed, containing a large amount of collagen, unlike mesenchymal stem cell spheroids, which do not actively secrete extracellular matrix. Therefore, in C-MSCs, the cell density is low and the percentage of collagen is high.

Cell density can be evaluated by staining a section of a periodontal tissue regeneration material with DAPI stain or the like to obtain a stained image, and then counting the number of cells present within an area of 150 µm x 150 µm of the stained image. In the periodontal tissue regeneration material of this embodiment, 50-300 cells, preferably 100-200 cells, and more preferably 120-180 cells, are present per 150 µm × 150 µm of the stained section image.

The percentage of collagen can be evaluated using, for example, a COL1-immunofluorescently stained section image. Specifically, in COL1-immunofluorescently stained section images of C-MSCs and a mesenchymal stem cell spheroid (a case where the spheroid is fabricated without culturing mesenchymal stem cells for promoting extracellular matrix secretion), the area fraction occupied by collagen in the section of C-MSCs is preferably 40% or more, more preferably 50% or more, and particularly preferably 60% or more. On the other hand, the area fraction of collagen in the section of the mesenchymal stem cell spheroid is lower than that of C-MSCs.

The upper limit of the area fraction occupied by collagen is preferably 90%, more preferably 85%, and particularly preferably 80%.

In the present invention, the cell density can also be evaluated by counting the number of cells present per one graft tissue. In the periodontal tissue regeneration material of this embodiment, 2 × 10⁵ cells, preferably 4 × 10⁵ cells, and more preferably 6 × 10⁵ cells are present per one graft tissue. Although not particularly limited, the number of cells per one graft tissue may be determined by selecting 10 to 50 or more graft tissue particles (C-MSCs), counting the number of cells in each individual graft tissue particle, and averaging the results.

The aforementioned collagen can also be evaluated by measuring the weight of collagen per one graft tissue. The periodontal tissue regeneration material of this embodiment contains 2 × 10⁵ ng or more of collagen, preferably 2.5 × 10⁵ ng or more of collagen, more preferably 3.0 × 10⁵ ng or more of collagen, and still more preferably 3.5×10⁵ ng or more of collagen per one graft tissue. Although not particularly limited, the weight of collagen per one graft tissue may be determined by selecting 10 to 50 or more graft tissue particles (C-MSCs), measuring the collagen content (weight) in each individual graft tissue particle, and averaging the results.

Furthermore, the periodontal tissue regeneration material of this embodiment preferably satisfies, as well as the above-mentioned weight of collagen per one graft tissue, the condition that the ratio (cells/ng) of the number of cells present in the graft tissue (cells) to the collagen content (ng) in the graft tissue (cells/ng) is 1.0-10.0, but the condition is not particularly limited thereto and the ratio (cells/ng) may be 1.6-6.0, 1.8-4.0, 2.0-3.0, or any combination of the upper and lower limits of these ratios.

Next, in step (b), a plurality of C-MSCs obtained above are used to fabricate a cellular construct. The number of C-MSCs may be determined according to the size of the cellular construct.

C-MSCs are arranged or molded to fabricate a cellular construct therefrom. Arrangement means to arrange C-MSCs in a one-dimensional (linear), two-dimensional (planar), or three-dimensional (three-dimensional) manner. Preferably, C-MSCs are arranged in a two- or three-dimensional manner, and more preferably, C-MSCs are arranged in a three-dimensional manner.

Arranging C-MSCs in a two- or three-dimensional manner is sometimes referred to herein as "stacking". When stacking C-MSCs, preferably a total of 3 or more C-MSCs, more preferably a total of 9 or more C-MSCs are arranged in the vertical, horizontal, and height directions. Although there is no particular upper limit to the number of stacked C-MSCs, the total number of C-MSCs is, for example, preferably 1,000 or less.

Known techniques for arranging C-MSCs include a method of fabricating a three-dimensional cellular construct by arranging cell aggregates in a predetermined three-dimensional space to stack them (WO2008/123614). According to this method, needle-like bodies are arranged on a substrate like a *Kenzan* (spiky flower frog), and cell aggregates are arranged in a three-dimensional manner by either inserting the needle-like bodies into the cell aggregates or piercing the cell aggregates with the needle-like bodies. A 3D bioprinter can be used for stacking. Examples of 3D bioprinter include S-PIKE manufactured by Cyfuse Biomedical K.K. and Regenova manufactured by Cyfuse Biomedical K.K.

The arranged or molded C-MSCs are cultured until the C-MSCs fuse with each other. In doing so, the medium preferably contains ascorbic acid or ascorbic acid phosphate ester. Thus, the periodontal tissue regeneration material of this embodiment is produced.

When producing the periodontal tissue regeneration material of this embodiment by the above-described production method, it is preferable that at least one selected from the group consisting of shaking culture, agitation culture, and no-shear agitated culture is performed at least once in the culture step. More preferably, at least the culture in step (b) is at least one selected from the group consisting of shaking culture, agitation culture, and no-shear agitated culture.

In the periodontal tissue regeneration material of this embodiment, extracellular matrix, such as collagen, is present in a large amount and mixed with mesenchymal stem cells. Therefore, in some embodiments, even without using an artificial scaffold material, it is possible to induce differentiation into periodontal tissue and realize regeneration of periodontal tissue by using collagen secreted by the cells as a self-scaffold, which is essentially composed of the cells and cell-derived extracellular matrix.

Regarding cell density, there are 50-300 cells, preferably 100-200 cells, and more preferably 120-180 cells, per 150 µm × 150 µm of the stained section image (e.g., DAPI-stained image) of the periodontal tissue regeneration material that is substantially free of artificial scaffold material.

When a section is cut out from the periodontal tissue regeneration material that is substantially free of artificial scaffold material and a COL1-immunofluorescently stained image is obtained to measure the area fraction of collagen, the area fraction occupied by collagen is preferably 40% or more, as described for the C-MSCs section above. More preferably, the area fraction of collagen is 50% or more, and particularly preferably 60% or more. While the upper limit of the area fraction is not particularly limited, it is preferably 90%, more preferably 85%, and particularly preferably 80%.

In addition, the periodontal tissue regeneration material of this embodiment can be made into any size or shape by adjusting the number and arrangement of C-MSCs. For example, the volume can be 1-500 mm³. The shape may be, for example, a roughly cubic shape or a roughly rectangular prism shape, or tubular (a hollow and elongated structure).

COL1 is preferred as the collagen.

The periodontal tissue regeneration material of this embodiment is a large cellular construct with a projected circular area-equivalent diameter or thickness of 1 mm or more, preferably 2 mm or more. Herein, a "projected circular area-equivalent diameter" refers to a circular-equivalent diameter of a projected area observed with an optical microscope and refers to the maximum value among the projected circular area-equivalent diameters obtained from all angles, which can be measured with a stereoscopic microscope. "Thickness" can also be measured in the same way.

Surprisingly, even though the periodontal tissue regeneration material of this embodiment is a large cellular construct, the cells residing inside it are viable, and preferably the cells residing inside are well nourished and healthy without quality deterioration. Specifically, 80% or more, 85% or more, 90% or more, or 95% or more of the total cells are positive for surface antigen markers CD29, CD44, CD73, CD90, CD105 and CD166 and negative for CD34 and CD45.

Because the size of the periodontal tissue regeneration material is large as mentioned above, it can be secured stably even in a large periodontal tissue defect site or in a periodontal tissue defect site with a shape that is hard to secure a periodontal tissue regeneration material (e.g., one-walled vertical bone defect, extensive horizontal bone defect, and dehiscence periodontal tissue defect, etc.).

Although the reason is unclear, it has been confirmed that mesenchymal stem cells and extracellular matrix, such as collagen, do not adhere to each other without gaps, and there are appropriate voids in the periodontal tissue regeneration material of this embodiment. In other words, the periodontal tissue regeneration material of this embodiment is porous. The quality of the cells inside appears to be maintained because these voids allow nutrients to be supplied inside.

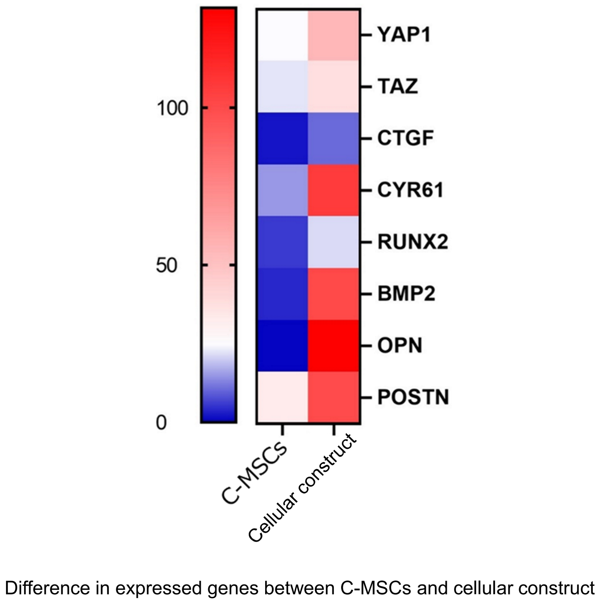

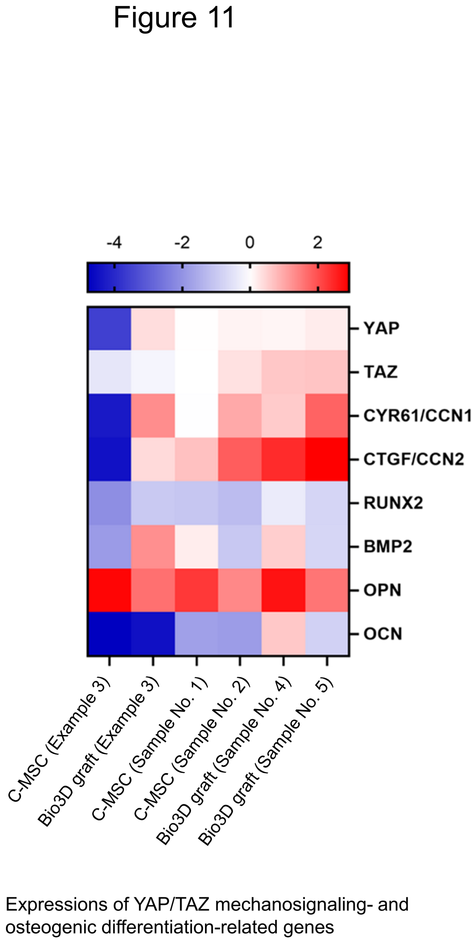

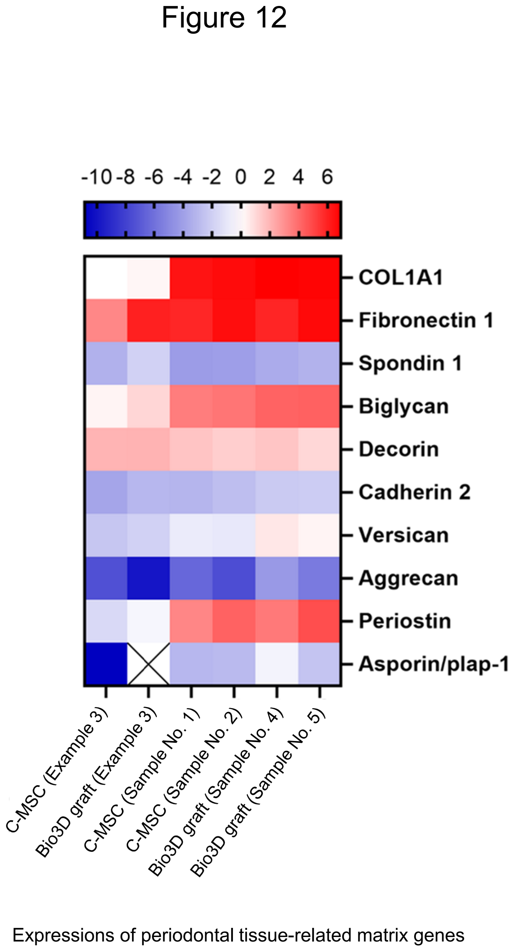

Furthermore, by making the periodontal tissue regeneration material of this embodiment a large cellular construct, the expression levels of a YAP/TAZ mechanosignaling-related gene cluster involved in the maintenance of bone/periodontal tissue homeostasis by stem cells tend to increase compared to those in the mesenchymal stem cells used as a material (before or in the state of C-MSCs). Preferably, the expression level of at least one YAP/TAZ mechanosignaling-related gene selected from the group consisting of YAP1, TAZ, CTGF, CYR61, RUNX2, BMP2, OPN, and POSTN is twice or more higher than that in the mesenchymal stem cells used as a material (see Figure 6 of this application). There is also a tendency for the expression levels of a matrix-producing gene cluster (bone differentiation-related gene cluster), which affects YAP/TAZ mechanosignaling and stiffness of the three-dimensional tissue, to increase. Specifically, the expression level of at least one gene selected from the group consisting of YAP1, TAZ, CYR61/CCN1, CTGF/CCN2, RUNX2, BMP2, OPN, and OCN is increased (see Figure 11 of this application). Furthermore, there is also a tendency for the expression levels of a periodontal tissue-related matrix gene cluster to increase. Specifically, the expression level of at least one gene selected from the group consisting of COL1A1, Fibronectin 1, Spondin 1, Biglycan, Decorin, Cadherin 2, Versican, Aggrecan, Periostin, and Asporin/plap-1 is increased (see Figure 12 of this application). These findings suggest that the periodontal tissue regeneration material of this embodiment may have a further enhanced ability to regenerate periodontal tissue while maintaining the basic structure and characteristics of C-MSCs.

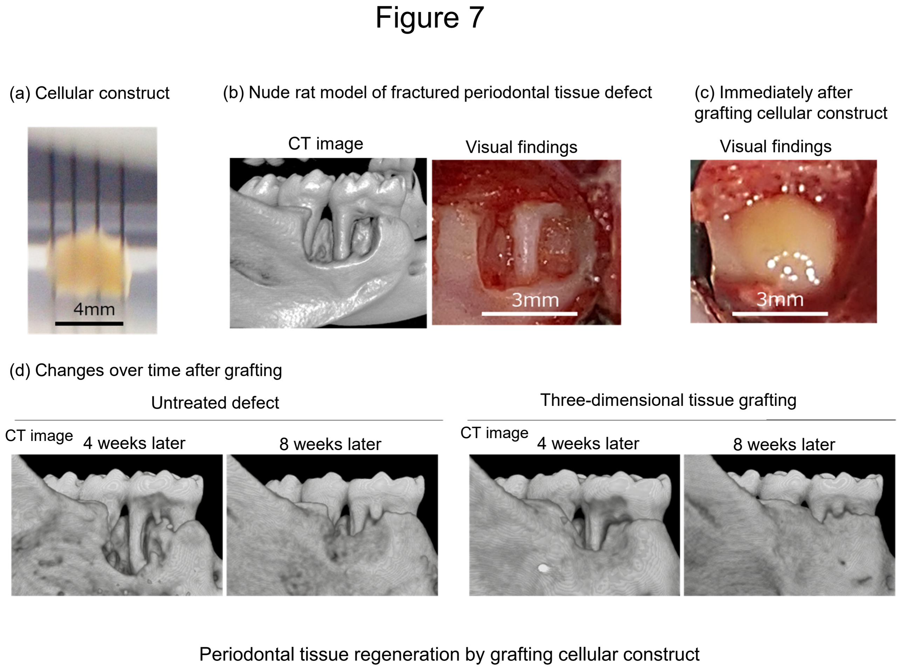

In fact, as shown in the examples below, when the periodontal tissue regeneration material of this embodiment was grafted into a one-walled, dehiscence periodontal tissue defect model, i.e., a nude rat with an alveolar bone defect, regeneration of periodontal tissue, including the alveolar bone, was confirmed (see, for example, Figures 7, 13, and 14 of this application).

### Grafting periodontal tissue regeneration material

The periodontal tissue regeneration material of this embodiment is grafted into a recipient patient (test animal). Examples of the recipient patient include patients with mild to severe periodontal disease.

The grafted site is not limited as long as the purpose is to regenerate periodontal tissue. The grafting method is also not particularly limited, and any method can be adopted.

It appears that when the periodontal tissue regeneration material of the present invention is grafted into a target site, the periodontal tissue regeneration material itself will differentiate into various periodontal tissue component cells, thereby regenerating tissue. Whether or not periodontal tissue has regenerated at the grafted site can be estimated in humans, for example, by means of CT examination, occlusal force, and periodontal pocket depth measurement.

### Other applications

So far, an example of a periodontal tissue regeneration material and a method for producing the same has been described, but the cellular construct for grafting obtained by the above-described production method can also be used for applications other than a periodontal tissue regeneration material. For example, it can be grafted into a patient with jawbone defect due to cancer. It can also be grafted into a patient who is about to undergo implant treatment.

### EXAMPLES

Hereinafter, the present invention will be described further in detail by way of examples. The present invention, however, should not be limited to these examples.

### [Example 1: Fabrication of cellular construct for grafting]

### (1) Fabrication of C-MSCs

Human bone marrow-derived mesenchymal stem cells (MSC, manufactured by LONZA) were seeded into a T225 flask at a density of 1.1 × 10⁶ cells/flask. DMEM low glucose + 10% FBS supplemented with 100 µg/ml streptomycin and 100 U/ml penicillin was used as the medium. The medium was changed every 2-3 days, and passages were performed once the culture reached subconfluence.

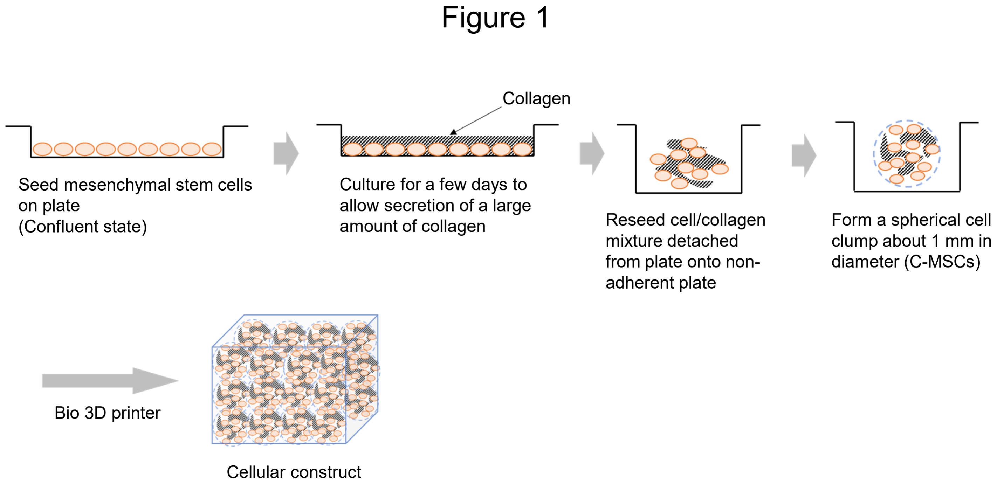

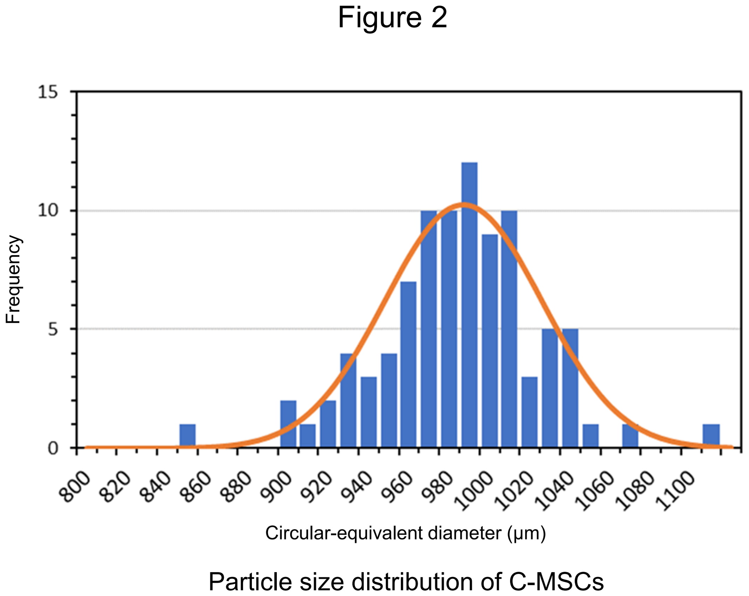

The above cells were detached and collected with trypsin/EDTA. As shown in Figure 1, the collected cells were seeded in a 24-well multi-well plate so that the number of cells was 2.0 × 10⁵ cells in each well. DMEM high Glucose + 10% FBS + 50 µg/ml ascorbic acid was used as the medium. The medium was changed on day 3. On day 4, the periphery of the wells was scratched with a micropipette tip to detach the cell sheets. The resulting sheets were aspirated with the micropipette tip and transferred to a 48-well ultra-low binding plate. Spherical C-MSCs were fabricated by culturing for 5 days using DMEM high glucose + 10% FBS + 50 µg/ml ascorbic acid as a medium. The sizes of the resulting C-MSCs are shown in Figure 2. The average size, standard deviation, and CV were 992 µm, 39 µm, and 4%, respectively. Particle sizes were measured using the measurement function of Regenova, a bio 3D printer manufactured by Cyfuse Biomedical K.K.

### (2) Fabrication of cellular construct

Using S-PIKE manufactured by Cyfuse Biomedical K.K. as a bio 3D printer, the fabricated C-MSCs were placed on needles to form a three-dimensionally stacked structure. The C-MSCs placed on the needles were aligned on a special stand to form a three-dimensionally stacked structure. Then, shaking culture (100 rpm) was performed continuously for 6 days to fuse and integrate the C-MSCs with each other, thereby fabricating a cellular construct. The medium contained L-ascorbic acid (concentration 50 µg/ml).

Similarly, cellular constructs of various shapes and sizes were fabricated. For example, for the grafting test in rats, graft tissue was fabricated using 16 (4 × 4) C-MSCs to obtain a cellular construct of 4 mm × 4 mm × 1 mm in size (Figure 7(a)).

### [Example 2: Characterization of cellular construct 1]

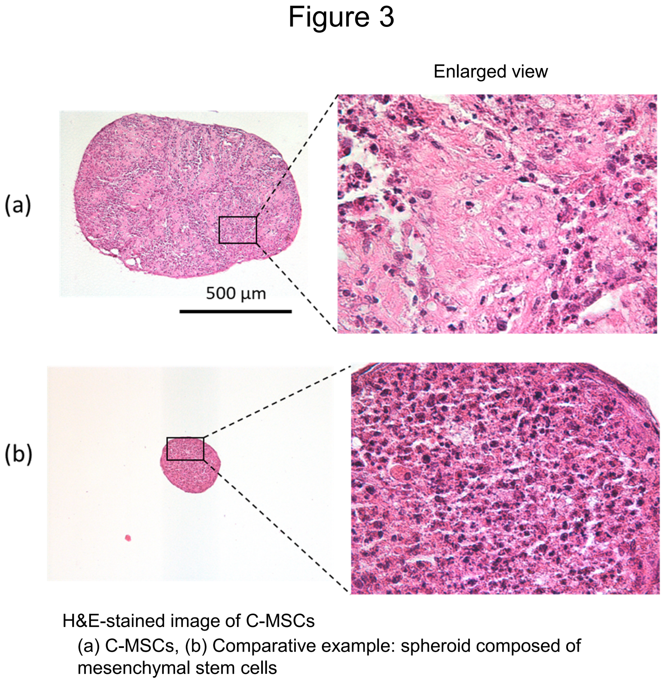

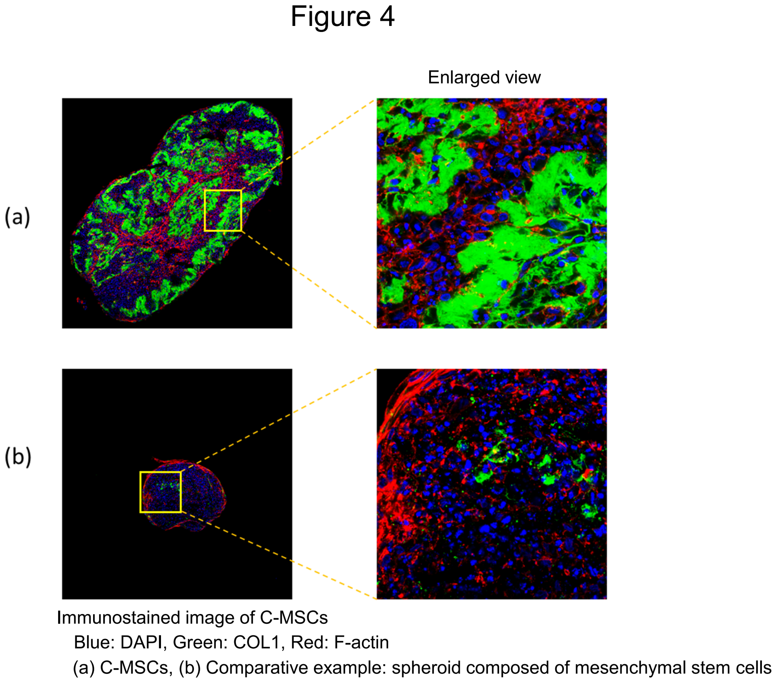

Histological analysis of the C-MSCs fabricated in Example 1 was performed. When a H&E-stained image of the C-MSCs was compared to a H&E-stained image of a spheroid composed of mesenchymal stem cells as a comparative example, the cell density of the C-MSCs was lower (Figure 3). Moreover, when DAPI-stained and COL1-immunostained images of the above C-MSCs were obtained and observed, a large amount of type I collagen existed inside the C-MSCs fabricated in Example 1, with the cells and type I collagen in a mixed state (Figure 4(a)). On the other hand, only a very small amount of type I collagen was observed inside the spheroid as the comparative example, and its structure was very different from that of the C-MSCs (Figure 4(b)).

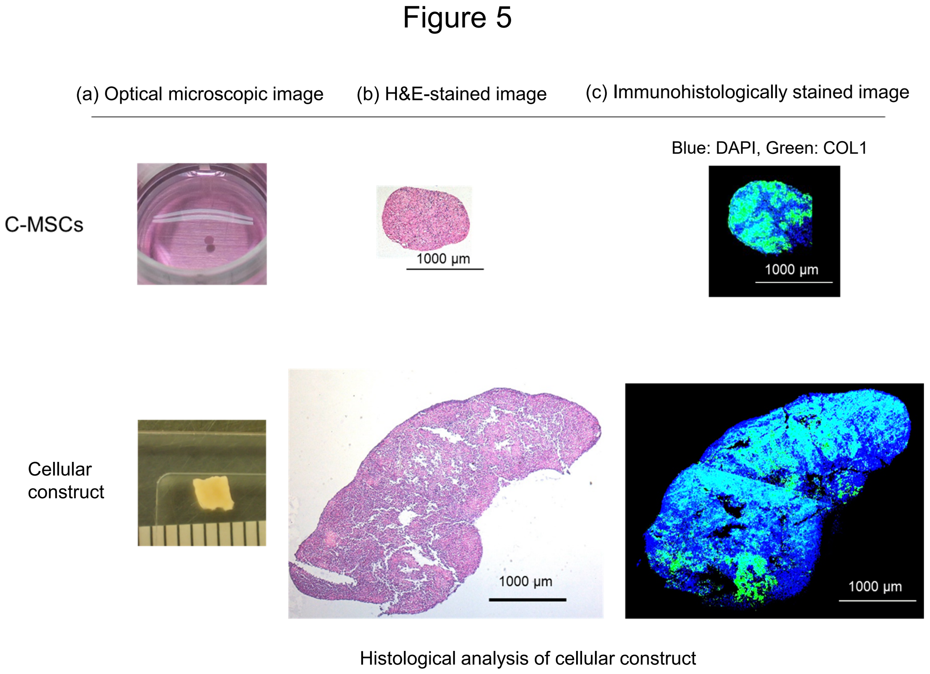

Next, the internal structure of the cellular construct fabricated by stacking the C-MSCs was analyzed (Figure 5). Even in a large cellular construct with a thickness exceeding 3 mm, cells in the center of the construct were viable. Similar to C-MSCs, a large amount of type I collagen and cells were in a mixed state in the cellular construct fabricated by stacking the C-MSCs.

The number of cells and the area fraction occupied by type I collagen, as measured from the obtained images, were as follows. The number of cells were determined by counting the number of stained particles in a 150 × 150 µm enlarged DAPI-stained image. The area fraction occupied by type I collagen was determined by counting the number of COL1-positive pixels in a COL1-immunostained image using ImageJ and dividing the number by the total number of pixels.

**[Table 1]**

| | Number of cells (cells/(150 µm × 150 µm)) | Area fraction occupied by type I collagen (%) |
|---|---|---|
| (Present invention) C-MSCs | 148 | 62 |
| (Present invention) Cellular construct | 138 | 66 |
| (Comparative example) Mesenchymal stem cell spheroid | 359 | 22 |

### [Example 3: Characterization of cellular construct 2]

Gene expressions in the C-MSCs and the cellular construct were comprehensively analyzed by the RNA-seq method. As a result, the expression levels of approximately 2,500 genes were twice or more higher in the cellular construct than those in the C-MSCs. Analysis of gene clusters with high expression levels revealed that a YAP/TAZ mechanosignaling-related (bone and periodontal tissue formation-related) gene cluster was highly expressed (Figure 6).

This suggests that the cellular construct of the present invention may have superior ability to regenerate bone and periodontal tissue through inherent mechanosignaling, while maintaining the basic structure of the C-MSCs. Characteristics such as extracellular matrix content, composition, and expressed genes may be used as new items of quality criteria for cellular constructs for grafting.

### [Example 4: Regeneration of periodontal tissue with cellular construct]

A one-walled, dehiscence periodontal tissue defect model, i.e., a nude rat with an alveolar bone defect, was prepared (Figure 7(b)). This was the most incurable rat model in which the defect site communicated with the oral cavity.

When a cellular construct (3 x 4 x 1 mm) was grafted into the defect site, the cellular construct was able to fill the entire defect site because of its flexibility (Figure 7(c)). X-ray micro-CT measurements were performed 4 and 8 weeks after grafting to evaluate alveolar bone formation (Figure 7(d)). In a sham-operated group without any graft at the defect site, there was very little regeneration at the defect site even after 8 weeks. On the other hand, bone formation had already progressed 4 weeks after grafting in the rat that was grafted with the cellular construct, and bone regeneration was found to be nearly complete 8 weeks after grafting.

Although not shown in the figure, little bone regeneration was observed in the group in which a plurality of C-MSCs were grafted.

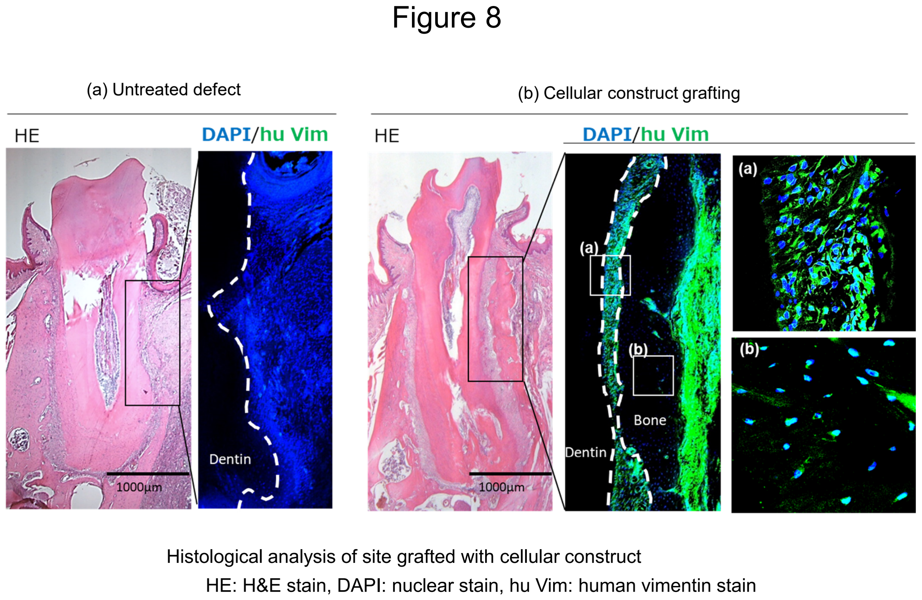

In addition, autopsies were performed 8 weeks after grafting and sections were cut out from the grafted site and the surrounding area to obtain H&E-stained and COL1-immunostained images. Furthermore, immunostaining using human-specific vimentin antibody was performed to examine the localization of the grafted human cells (Figure 8). As a result, in addition to alveolar bone regeneration, cementum and periodontal ligament regeneration on the root surface were confirmed at the grafted site, indicating regeneration of periodontal tissue. In addition, a significant number of human vimentin-positive cells were confirmed in the new periodontal tissue, indicating that the grafted mesenchymal stem cells differentiated into cells that form periodontal tissue and that tissue regeneration progressed.

The mechanism of curing using the cellular construct of the present invention was shown to be that the grafted mesenchymal stem cells themselves differentiated into various periodontal tissue component cells while using COL1, the major periodontal tissue ECM secreted by the C-MSCs themselves, as a scaffold, thereby achieving tissue reconstruction.

These results also correlated with the gene expression results shown in Example 3.

Thus, the large cellular construct fabricated from the C-MSCs and containing substantially no artificial material has tissue regeneration functions that are equivalent to or superior to those of the C-MSCs, and is capable of forming cell clumps into a single cohesive mass at a large defect site. Therefore, it is possible to regenerate tissue, for example, even at sites with severe periodontal tissue defects, including the most severe one-walled, horizontal bone defects among the patients with severe periodontitis.

### [Example 5: Fabrication of construct for grafting]

### (1) Fabrication of C-MSCs

Human bone marrow-derived mesenchymal stem cells (BMSC, manufactured by LONZA) or human adipose-derived mesenchymal stem cells (ADSC, manufactured by LONZA) were seeded into a T225 flask at a density of 1.1 × 10⁶ cells/flask. In doing so, PRIME-XV MSC XSFM MDF1 manufactured by Irvine (FUJIFILM Irvine Scientific, Inc.) was used as the medium. The medium was changed every 2-3 days, and passages were performed once the culture reached subconfluence.

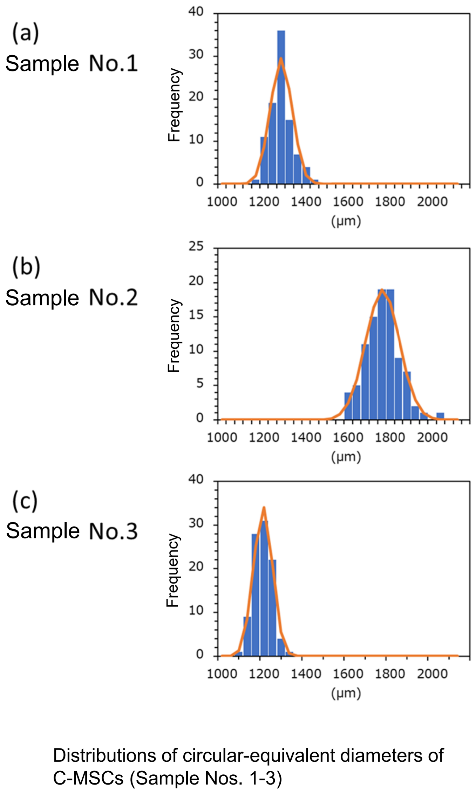

The above cells were detached and collected with trypsin/EDTA. The collected cells were seeded in a 48-well multi-well plate so that the number of cells was 1.0 × 10⁵ cells in each well. PRIME-XV MSC XSFM MDF1 manufactured by Irvine was used as the medium. On day 2 or day 7, the periphery of the wells was scratched with a micropipette tip to detach the cell sheets. The resulting sheets were aspirated with the micropipette tip and transferred to a 48-well ultra-low binding plate (ECM production step). Spherical C-MSCs were fabricated by culturing for 2 or 5 days using PRIME-XV MSC XSFM MDF1 manufactured by Irvine as a medium (C-MSC production step). Fabrication conditions and particle sizes (circular-equivalent diameters) of C-MSCs are shown in Table 2. Distribution of the circular-equivalent diameters of the C-MSCs for each sample is shown in Figure 9. Particle sizes were measured using the measurement function of Regenova, a bio 3D printer manufactured by Cyfuse Biomedical K.K.

**[Table 2]**

| Sample No. | Cell type | Schedule of steps (days) | | Circular-equivalent diameter (µm) | Standard deviation (µm) |
|---|---|---|---|---|---|
| | | Production of ECM | Fabrication of C-MSCs | | |
| No. 1 | BMSC | 2 | 5 | 1,298 | 52 |
| No. 2 | BMSC | 7 | 5 | 1,783 | 81 |
| No. 3 | ADSC | 2 | 2 | 1,215 | 45 |

### (2) Fabrication of cellular construct

Regenova manufactured by Cyfuse Biomedical K.K. was used as the bio 3D printer. The fabricated C-MSCs were placed on special *Kenzan-*like needles to give a predesigned three-dimensional shape, thereby fabricating a three-dimensionally stacked structure. Then, shaking culture (100 rpm) was performed continuously for 8 days (maturation step) to fuse and integrate the C-MSCs with each other, thereby fabricating a cellular construct. Cellular constructs of various shapes and sizes were fabricated by this method. For example, for the grafting test in rats, graft tissue with a total of 16 C-MSCs (4 vertically and 4 horizontally) arranged in a plane, was fabricated to obtain a cellular construct of 3 mm × 4 mm × 1 mm in size.

**[Table 3]**

| 3D graft tissue Sample No. | Sample No. of C-MSCs used for stacking | Number of stacks | Schedule of maturing step (Day) | Size of graft tissue (mm) |
|---|---|---|---|---|
| No. 4 | No. 1 | 4 × 4 | 8 | 2.4 × 3.0 × 1.0 |
| No. 5 | No. 2 | 4 × 4 | 8 | 3.0 × 3.5 × 1.0 |
| No. 6 | No. 3 | 4 × 4 | 8 | 2.8 × 2.5 × 1.0 |

### [Example 6: Characterization of cellular construct 1]

The number of viable cells and the collagen content in the graft tissue fabricated in Example 5 were determined. The graft tissue was dispersed into single cells by immersing it in a collagenase solution. This solution was stained with 4',6-diamidino-2-phenylindole, dihydrochloride (DAPI), and acridine orange (AO) to count the total number of cells and dead cells. The number of viable cells contained in the graft tissue was calculated by subtracting the number of dead cells from the total number of cells.

Enzymatic degradation treatment of the graft tissue was performed using a commercially available collagen quantification kit. Since collagen produces a large amount of N-terminal glycine-containing fragments in doing so, these fragments were fluorescently derivatized to calculate the collagen concentration and the collagen content in the graft tissue from the measurement of the fluorescence intensity of the solution. The ratio of the number of viable cells to the collagen content determined above was calculated as Cell/Col ratio (cells/ng). These results are summarized in Table 4.

**[Table 4]**

| Sample No. | Number of viable cells 10⁵ (cells/tissue) | Collagen content 10⁵ (ng/tissue) | Cell/Col |
|---|---|---|---|
| No. 4 | 9.5 | 2.9 | 3.3 |
| No. 5 | 11.4 | 6.7 | 1.7 |
| No. 6 | 12.2 | 5.1 | 2.4 |

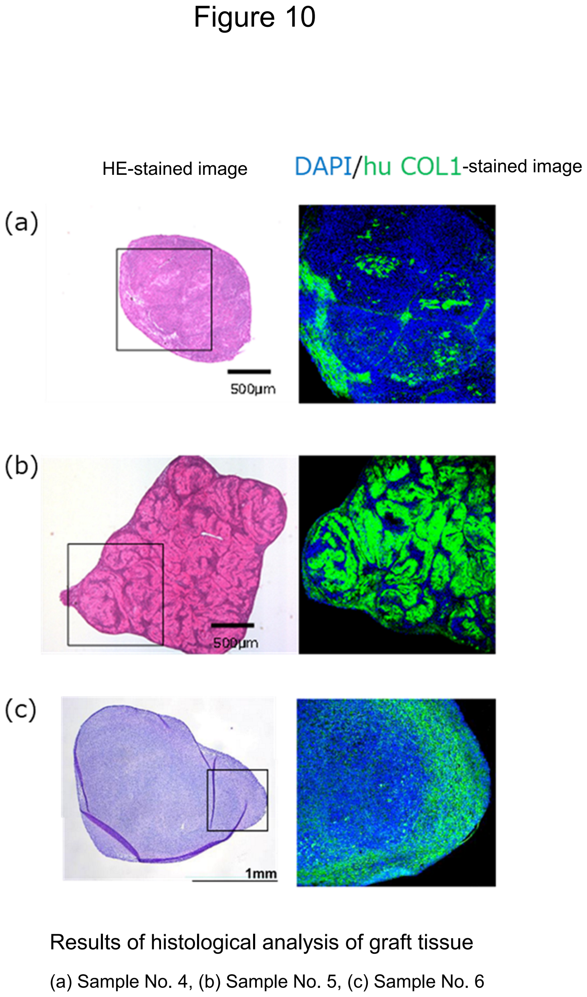

Sections of these samples were prepared and subjected to HE staining and DAPI/COL1 staining. The results are shown in Figure 10. Collagen was observed throughout Sample No. 5, which had a high collagen content. Sample No. 6, which used ADSC as a source, was found to have a characteristic structure with a large amount of collagen at the periphery of the three-dimensional tissue.

### [Example 7: Characterization of cellular construct 2]

Gene expressions in the C-MSCs and the cellular construct were comprehensively analyzed by the RNA-seq method. As a result, fluctuation in gene expressions were observed in 1,867 genes in Sample No. 1 (C-MSCs) and the three-dimensional graft tissue fabricated from the C-MSCs (Sample No. 4). Analysis of gene clusters with high expression levels revealed enhanced expression of the matrix-producing gene cluster, which affects YAP/TAZ mechanosignaling and the stiffness of three-dimensional tissue (Figure 11). Additionally, when compared to Example 3, the sample fabricated in Example 5 tended to show enhanced YAP/TAZ gene expressions in both the C-MSCs and the three-dimensional graft tissue. In particular, the three-dimensional graft tissue (Sample No. 4) showed markedly enhanced expressions of RUNX2 (transcription factor essential for bone formation), BMP2 (which induces bone formation and regeneration), OPN (important factor for bone remodeling), and OCN (which is secreted in large amount by mature osteoblasts, and is involved in calcification of extracellular matrix and formation of rigid bone matrix), suggesting that it has cell properties suitable for bone regeneration.

In addition, focusing on the expressions of periodontal tissue-related matrix genes, the C-MSCs and the three-dimensional graft tissue fabricated in Example 5 expressed a cluster of genes that were excellent for periodontal tissue reconstruction, including COL1 and periodontal ligament matrix proteins (biglycan and versican) (Figure 12). These results confirmed that graft tissue fabricated by three-dimensionally stacking the C-MSCs using a bio 3D printer expresses a cluster of genes advantageous for periodontal tissue regeneration.

### [Example 8: Regeneration of periodontal tissue with cellular construct]

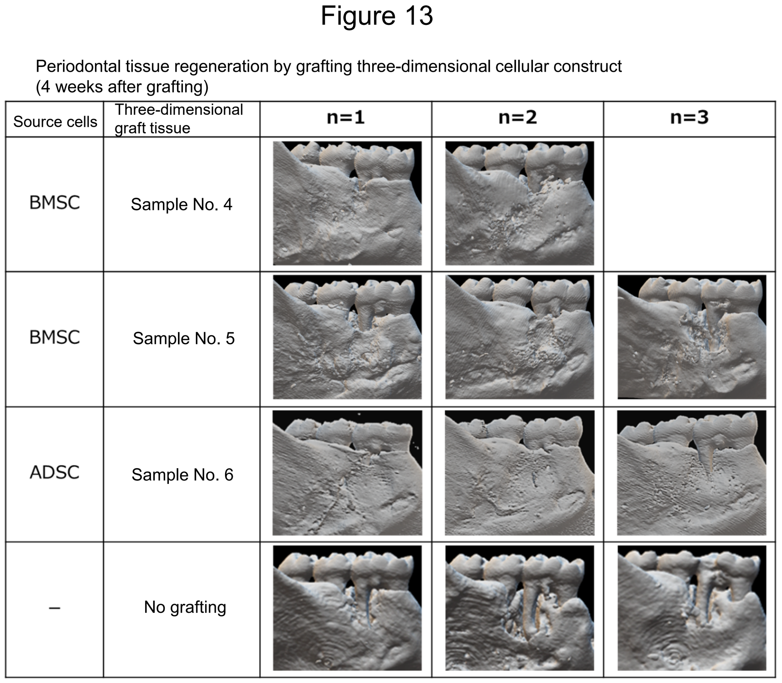

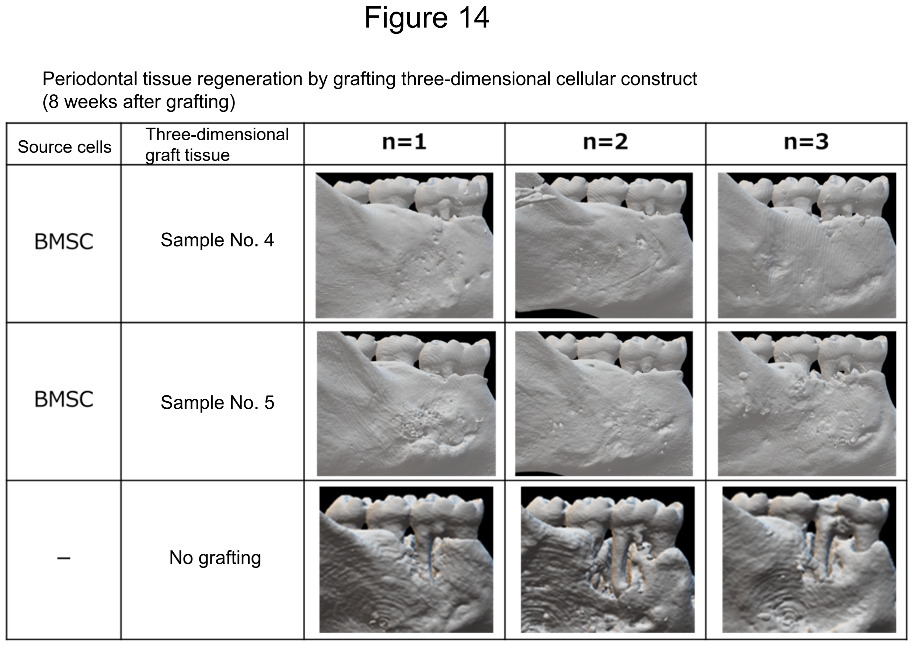

The cellular constructs (3 × 4 × 1 mm) of Sample Nos. 4, 5, and 6 were grafted into the defect sites of one-walled, dehiscence periodontal tissue defect models, i.e., nude rats with alveolar bone defects. The cellular constructs were able to fill the entire defect sites because of their flexibility. X-ray micro-CT measurements were performed 4 and 8 weeks after grafting to evaluate alveolar bone formation (Figures 13 and 14). In a sham-operated group without any graft at the defect site, there was very little bone regeneration at the defect site even after 8 weeks. In contrast, bone formation had already progressed significantly in rats grafted with the BMSC-derived cellular constructs (Sample Nos. 4 and 5) 4 weeks after grafting. The extent of bone formation was better in Sample No. 4. By 8 weeks after grafting, bone formation was nearly complete in both Sample Nos. 4 and 5. Furthermore, progress in bone formation was remarkable when the ADSC-derived cellular construct (Sample No. 6) was grafted, resulting in nearly complete bone regeneration 4 weeks after grafting. These results showed that the bone formation rate of the graft tissue fabricated in Example 5 was higher than that in Example 3, and alveolar bone regeneration was achieved in a short period of time.

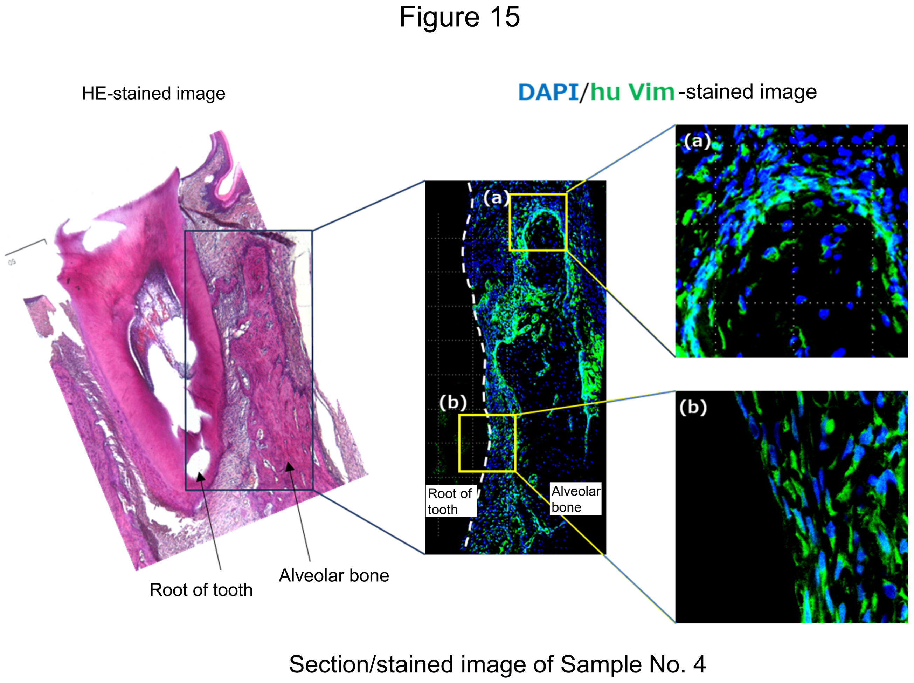

Autopsies were performed 8 weeks after grafting and sections were cut out from the grafted site and the surrounding area to obtain H&E-stained and COL1-immunostained images. Immunostaining using human-specific vimentin antibody was also performed to examine the persistence and localization of human cells at the grafted site (Figure 15). As a result, in addition to alveolar bone regeneration, cementum was observed covering the root surface and connected to the alveolar bone via the periodontal ligament at the grafted site, indicating regeneration of periodontal tissue. A significant number of human vimentin-positive cells were also observed in the area corresponding to the new cementum and periodontal ligament. Human vimentin-positive cells were also observed on the surface and inside the new bone in a greater number than the negative cells. It is highly likely that the grafted human cells have differentiated into osteoblasts and then into osteocytes, and the mechanism of action of the three-dimensional graft tissue of the present invention is considered to be tissue reconstruction by the grafted cells.

### INDUSTRIAL APPLICABILITY

The present invention can provide a periodontal tissue regeneration material and the like that can be grafted into a site of severe periodontal tissue defect to achieve tissue regeneration at the grated site.

## Claims

1. A periodontal tissue regeneration material for grafting, comprising graft tissue containing mesenchymal stem cells and collagen secreted by the mesenchymal stem cells, wherein
the graft tissue each has 2 × 10⁵ or more cells and has a projected circular area-equivalent diameter or thickness of 1 mm or more.

2. The periodontal tissue regeneration material according to claim 1, wherein the graft tissue each contains 2 x 10⁵ ng or more of collagen, and the ratio of the number of cells present in the graft tissue (cells) to the collagen content in the graft tissue (ng) (cells/ng) is 1.0-10.0.

3. The periodontal tissue regeneration material according to claim 1, wherein the graft tissue each has 4 × 10⁵ or more cells and has a projected circular area-equivalent diameter or thickness of 1 mm or more.

4. The periodontal tissue regeneration material according to claim 2, wherein the graft tissue each contains 2.5 × 10⁵ ng or more of collagen, and the ratio (cells/ng) is 1.6-6.0.

5. A periodontal tissue regeneration material for grafting, comprising graft tissue containing mesenchymal stem cells and collagen secreted by the mesenchymal stem cells, wherein
the graft tissue has 50-300 cells per 150 µm × 150 µm of a stained section image and has a projected circular area-equivalent diameter or thickness of 1 mm or more.

6. The periodontal tissue regeneration material according to claim 5, wherein the graft tissue has an area fraction occupied by collagen of 40% or more in a COL1-immunohistologically stained section image.

7. The periodontal tissue regeneration material according to claim 1 or 5, wherein 80% or more of the total cells in the graft tissue are positive for surface antigen markers CD29, CD44, CD73, CD90, CD105 and CD166, and negative for CD34 and CD45.

8. The periodontal tissue regeneration material according to claim 1 or 5, wherein the periodontal tissue regeneration material has a volume of 1-500 mm³.

9. The periodontal tissue regeneration material according to claim 1 or 5, wherein the periodontal tissue regeneration material has a roughly cubic shape or a roughly rectangular prism shape.

10. The periodontal tissue regeneration material according to claim 1 or 5, wherein the periodontal tissue regeneration material is tubular.

11. The periodontal tissue regeneration material according to claim 1 or 5, wherein the mesenchymal stem cells are at least one selected from the group consisting of bone marrow-derived mesenchymal stem cells, adipose-derived mesenchymal stem cells, placenta-derived mesenchymal stem cells, human iPS cell-derived mesenchymal stem cells, and human ES cell-derived mesenchymal stem cells.

12. The periodontal tissue regeneration material according to claim 1 or 5, wherein the expression level of at least one YAP/TAZ mechanosignaling-related gene selected from the group consisting of YAP1, TAZ, CTGF, CYR61, RUNX2, BMP2, OPN, and POSTN is twice or more higher than that of the mesenchymal stem cells used as a material.

13. The periodontal tissue regeneration material according to claim 1 or 5, wherein the collagen is COL1.

14. A method for producing a cellular construct for grafting, comprising the steps of:
(a) fabricating cell clumps (C-MSCs) using mesenchymal stem cells and extracellular matrix secreted by the mesenchymal stem cells; and
(b) arranging or molding the C-MSCs to fabricate the cellular construct for grafting.

15. The method according to claim 14, wherein, in steps (a) and (b) above, a medium containing ascorbic acid or ascorbic acid phosphate ester is used.

16. The method according to claim 14, wherein the C-MSCs have an average particle size of 600-2,000 µm.

17. The method according to claim 16, wherein the C-MSCs have an average particle size of 800-1,500 µm.

18. The method according to claim 17, wherein the C-MSCs have an average particle size of 900-1,200 µm.

19. The method according to claim 14, wherein in steps (a) and/or (b), shaking culture, agitation culture, or no-shear agitation culture is performed.

20. A cellular construct for grafting, which is produced by the method according to claim 14.

21. The cellular construct for grafting according to claim 20, which is used as a periodontal tissue regeneration material.
